# EUROPEAN PATENT APPLICATION

(11) **EP 4 474 375 A1**
(43) Date of publication of application: **11.12.2024**
(21) Application number: 23749830.8
(22) Date of filing: 02.02.2023
(51) Int. Cl.: C07C 51/00, C07C 55/14, C07C 209/62, C07C 211/12, C08G 69/26, C12N 9/80, C12P 1/00, C12P 7/44, C12P 13/00

(54) **METHOD FOR PRODUCING DICARBOXYLIC ACID COMPOUND AND/OR DIAMINE COMPOUND USING POLYAMIDE AS STARTING MATERIAL**

(30) Priority: 02.02.2022 JP 2022014609
(71) Applicant: Asahi Kasei Kabushiki Kaisha, Tokyo 1000006 (JP)
(72) Inventor: ARAMAKI Masaaki, Tokyo 100-0006 (JP); IWASE Katsuhiro, Tokyo 100-0006 (JP); ISAKA Koji, Tokyo 100-0006 (JP); MIYATAKE Ryo, Tokyo 100-0006 (JP); YAKIGAYA Kenichi, Tokyo 100-0006 (JP); OTANI Shoji, Tokyo 100-0006 (JP); YAMADA Yutaro, Tokyo 100-0006 (JP)
(74) Representative: D Young & Co LLP
(86) International application number: PCT/JP2023/003434
(87) International publication number: WO 2023/149514

(57) **Abstract**

The present disclosure relates to a production method of a dicarboxylic acid compound and/or diamine compound, and optionally, dehydration condensates of the dicarboxylic acid compound and diamine compound (dehydration condensate of one molecule of the dicarboxylic acid compound and one molecule of the diamine compound and dehydration condensates of one molecule of one of these and two molecules of the other), from a polyamide as the raw material. It includes (i) a first hydrolysis step of hydrolyzing the polyamide in high-temperature water to obtain a first hydrolysate, and (ii) a second hydrolysis step of subjecting the first hydrolysate to enzymatic hydrolysis to obtain a second hydrolysate. The first hydrolysate contains a water-soluble polyamide that dissolves in water at 20 °C, and the second hydrolysate contains the dicarboxylic acid compound and/or diamine compound, and optionally contains a dehydration condensate of the dicarboxylic acid compound and diamine compound.

## Description

### TECHNICAL FIELD

The present disclosure relates to a production method of a dicarboxylic acid compound and/or a diamine compound from a polyamide as the raw material. More particularly, the present disclosure relates to a production method of a dicarboxylic acid compound and/or a diamine compound, and optionally, dehydration condensates of the dicarboxylic acid compound and the diamine compound (dehydration condensate of one molecule of the dicarboxylic acid compound and one molecule of the diamine compound, and dehydration condensates of one molecule of one of these and two molecules of the other), from a polyamide as the raw material.

### BACKGROUND

Polyamides such as Nylon 6 and Nylon 66 are representative engineering plastics. They have high heat resistance and excellent mechanical properties, and are widely used in various applications such as fibers, automotive parts, and parts for electrical products. They are considered indispensable materials in modern society.

In recent years, various technologies have been developed for plastic recycling with the aim of resource conservation and carbon neutrality, and polyamides are no exception.

Recycling is roughly classified into two types: material recycling, which involves re-pelletizing products that have been shaped, and chemical recycling, which involves reusing monomers through depolymerization. Material recycling, although relatively straightforward, raises concerns due to the degradation of polymers in shaped products and the retention of additives in the recycled polymer, leading to unstable quality. On the other hand, in chemical recycling, since polymers are reverted back to monomers, degradation can be avoided. Additionally, high-purity monomers, free from contaminants, can be obtained and used as raw materials to produce the desired polymers.

A depolymerization reaction which is the fundamental process in chemical recycling depends on the bonding pattern of the polymer and the stability of the monomers that constitute the polymer. The amide bond constituting the main chain of polyamide is formed through a dehydration condensation between an amine and a carboxylic acid. Conversely, the depolymerization reaction, the reverse reaction, occurs through a hydrolysis reaction. Methods for hydrolysis reactions include reactions under acidic or basic conditions, as well as reactions using high-temperature water, supercritical water, or steam. Polyamides exhibit high thermal stability, necessitating high-temperature reactions under any reaction conditions. Therefore, the stability of the monomers under these reaction conditions significantly impacts the efficiency of chemical recycling.

Polyamides are classified into two types based on their constituent monomers. One type is n-nylon (such as Nylon 6), which is derived from amino acids as raw materials. The other type is n,m-nylon (such as Nylon 66), obtained through copolymerization reactions of monomers, namely, diamines and dicarboxylic acids.

When n-nylon is depolymerized via hydrolysis, amino acids are produced. When heated in water, typically, n-nylon undergoes cyclization via an equilibrium reaction to produce lactam. Yet, since lactam *per se* is a monomer for n-nylon polymerization, monomers can be obtained at a high yield.

On the other hand, n,m-nylon such as Nylon 66, when subjected to hydrolysis, produces diamines and dicarboxylic acids. However, these compounds are generally prone to undergo cyclization reactions when heated in water. In particular, the linear diamine compounds commonly used in typical nylon are prone to and irreversibly undergo cyclization reactions easily, making their recovery difficult. For instance, hexamethylenediamine decomposes during hydrolysis in high-temperature water and is thus undetected (PTL 1). Additionally, due to the progress of cyclization reactions, the yield of adipic acid has only improved to around 25%, rendering it insufficient for industrial-scale production applications.

To suppress the cyclization reactions of diamine compounds and dicarboxylic acid compounds, it is necessary to conduct the reaction at low temperatures. Although this is typically difficult with conventional organic chemical reactions, enzymatic hydrolysis reactions have been proposed as a solution. For example, a method using enzymes to hydrolyze Nylon 6,6 has been proposed (NPL 1). However, polyamides also contain crystallized regions formed by strong hydrogen bonds between amide bonds. Therefore, at low temperatures, it is difficult for the polymer chains to interact with the active sites of enzymes, resulting in very slow progress of the reaction. For example, in the hydrolysis of Nylon 6,6 using enzymes as described in the above example (NPL 1), the yield of enzymatic degradation of powdered Nylon 6,6 was only around 10%, even when combined with soluble oligomers. Furthermore, although enzyme reactions exhibit rapid initial reaction rates, the reaction rates significantly decrease afterward. This is presumably because the relatively high reactivity of the amorphous regions of the polymer surface finishes reacting in the initial stages, leaving only the less reactive crystalline regions.

A method proposed to enhance reactivity is to copolymerize components that reduce crystallinity (NPL 1). However, this approach reduces the inherent functionality of polymers, resulting in limited versatility.

Furthermore, a method proposed to enhance solubility before enzymatic reactions is to reduce the molecular weight, specifically by decomposing Nylon 6 with formic acid prior to enzyme reaction (NPL 2). However, when n,m-nylon is decomposed by formic acid, diamine compounds react with formic acid, thereby forming formic acid amide compounds. Even if the formic acid amide compounds are hydrolyzed, dicarboxylic acid compounds and diamine compounds can only be obtained as a mixture with formic acid. If formic acid remains, it contributes to the formation of monocarboxylic acids at the polymer ends during polymerization, making it difficult to increase the molecular weight of the polymer. Due to the significant impact even with small amounts remaining, monocarboxylic acid compounds such as formic acid need to be thoroughly removed. However, removal thereof is extremely difficult because they form salts with diamine compounds.

### CITATION LIST

### Patent Literature

PTL 1: JP 2001-302597 A

### Non-patent Literature

NPL 1:
   Nagai K, Iida K, Shimizu K, Kinugasa R, Izumi M, Kato D, Takeo M, Mochiji K, Negoro S., Appl Microbiol Biotechnol. 98(20): 8751-61 (2014)
NPL 2:
   Negoro S, Kato DI, Ohki T, Yasuhira K, Kawashima Y, Nagai K, Takeo M, Shibata N, Kamiya K, Shigeta Y, Methods Enzymol.648, 357-389 (2021)

### SUMMARY

### (Technical Problem)

An object of the present disclosure is to provide a novel method of producing a diamine compound and/or a dicarboxylic acid compound, which are raw monomers for polymerization, from a polyamide.

### (Solution to Problem)

The present inventors considered enzymatic hydrolysis reactions at low temperatures preferable as a method to minimize the loss of target products due to side reactions. To enhance hydrolysis reactivity by enzymes, reducing the molecular weight and solubilizing polyamides in water is effective, as demonstrated in the example of PA6 disclosed in NPL 1. However, NPL 1 fails to disclose the reduction of the molecular weight of n,m-nylon, and recovery of the target products in high purity has been extremely difficult with the decomposition method used for Nylon 6.

The present inventors have conducted various studies on methods for reducing the molecular weight of n,m-nylon prior to enzymatic degradation. As a result, we have discovered that the hydrolysis method in high-temperature water, which was conventionally avoided due to the decomposition reaction of the products, was optimal. Conventionally, studies were conducted from the viewpoint of increasing the yield of dicarboxylic acid by conducting hydrolysis in high-temperature water. We, on the other hand, have conducted studies aimed at suppressing the decomposition reaction of monomers during the stage of high-temperature hydrolysis, as well as obtaining low molecular weight products prone to enzymatic reactions. As a result, we have discovered that optimizing conditions, including enzymatic reactions, significantly improves the final yields of the production method, thereby completing the present disclosure.

Specifically, the present disclosure provides the following.
[1] A production method of a dicarboxylic acid compound (2) represented by the following general formula (2) and/or a diamine compound (3) represented by the following general formula (3), and optionally, a salt compound (4) represented by the following general formula (4), a polyamide (5) represented by the following general formula (5), and/or a polyamide (6) represented by the following general formula (6), from a polyamide (1) represented by the following general formula (1), the method comprising:
   (i) a first hydrolysis step of hydrolyzing the polyamide (1) in high-temperature water to obtain a first hydrolysate; and
   (ii) a second hydrolysis step of subjecting the first hydrolysate to an enzymatic hydrolysis to obtain a second hydrolysate,
      wherein the first hydrolysate contains a water-soluble polyamide that dissolves in water at 20 °C, and
      the second hydrolysate contains the dicarboxylic acid compound (2) and/or the diamine compound (3) and, optionally, the salt compound (4), the polyamide (5), and/or the polyamide (6): 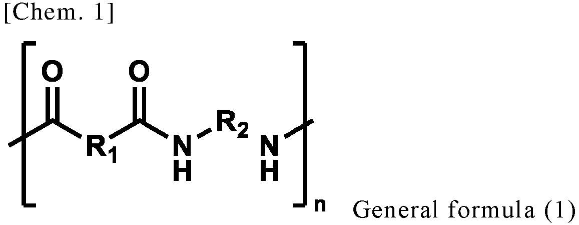 in the formula,
      R₁ is a substituted or unsubstituted aliphatic or aromatic hydrocarbon group having 1 to 10 carbons, and
      R₂ is a substituted or unsubstituted aliphatic hydrocarbon group having 2 to 10 carbons, 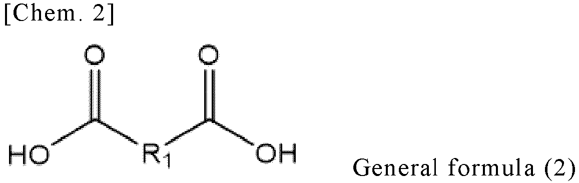 in the formula, R₁ is as defined in the general formula (1), 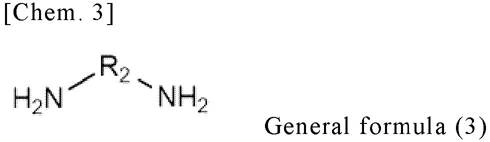 in the formula, R₂ is as defined in the general formula (1), 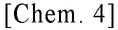 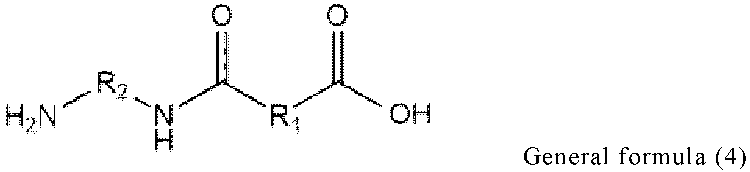 in the formula, R₁ and R₂ are as defined in the general formula (1), 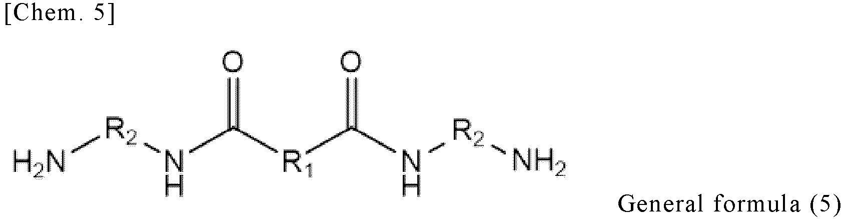 in the formula, R₁ and R₂ are as defined in the general formula (1), 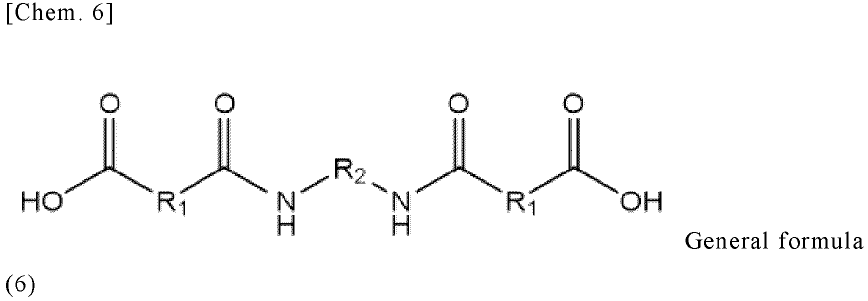 in the formula, R₁ and R₂ are as defined in the general formula (1).
[2] The production method according to [1], wherein
   the dicarboxylic acid compound (2) represented by the general formula (2) and the diamine compound (3) represented by the general formula (3) are produced from the polyamide (1) represented by the general formula (1), and
   the second hydrolysate contains the dicarboxylic acid compound (2) and the diamine compound (3).
[3] The production method according to [1] or [2], wherein a temperature of the high-temperature water is lower than a critical temperature of the water.
[4] The production method according to any one of [1] to [3], wherein in the first hydrolysis step, a pH of a hydrolysis reaction solution at the end of hydrolysis is from 7.0 to 10.0 when a concentration of the polyamide (1) represented by the general formula (1) is 10 weight% relative to 100 weight% of the high-temperature water.
[5] The production method according to any one of [1] to [4], wherein
   the first hydrolysate further contains a polyamide that is insoluble in water at 20 °C, and
   a number average molecular weight of the insoluble polyamide is 5000 or less.
[6] The production method according to any one of [1] to [5], wherein
   the first hydrolysate further contains the diamine compound (3) represented by the general formula (3) and a cyclic compound (7) represented by the following general formula (7), and
   a sum of yields of the diamine compound (3) represented by the general formula (3) and the cyclic compound (7) represented by the following general formula (7) is 20% or less: 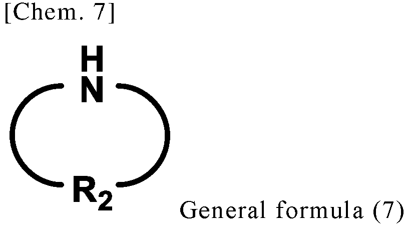 in the formula, R₂ is as defined in the general formula (1).
[7] The production method according to any one of [1] to [6], wherein R₂ is a substituted or unsubstituted aliphatic hydrocarbon group having 5 to 9 carbons.
[8] The production method according to any one of [1] to [7], wherein R₁ is an unsubstituted linear aliphatic or aromatic hydrocarbon group having 4 to 8 carbons.
[9] The production method according to any one of [1] to [8], wherein R₂ is an unsubstituted linear aliphatic hydrocarbon group having 6 carbons.
[10] The production method according to any one of [1] to [9], wherein R₁ is an unsubstituted linear aliphatic hydrocarbon group having 4 carbons.

### (Advantageous Effect)

In accordance with the present disclosure, a novel method of producing a dicarboxylic acid compound and/or a diamine compound, which are raw materials for polymerization, from a polyamide is provided.

### BRIEF DESCRIPTION OF THE DRAWINGS

[FIG. 1] FIG. 1 is a diagram depicting the amino acid sequences of enzymes and the base sequences of those enzymes with codon optimization for expression in *Escherichia coli*; and
[FIG. 2] FIG. 2 is a diagram depicting base sequences of primers.

### DETAILED DESCRIPTION

The following provides a detailed description of an embodiment of the present disclosure (hereinafter, referred to as the "present embodiment"). In this specification, nucleotide sequences are described in the 5' to 3' direction, and amino acid sequences are described in the N-terminal to C-terminal direction unless otherwise stated herein.

The present disclosure relates to a production method of a dicarboxylic acid compound (2) represented by the following general formula (2) and/or a diamine compound (3) represented by the following general formula (3), and optionally, a salt compound (4) represented by the following general formula (4), a polyamide compound (5) represented by the following general formula (5), and/or a polyamide compound (6) represented by the following general formula (6), from a polyamide (1) represented by the following general formula (1), the method including:
(i) a first hydrolysis step of hydrolyzing the polyamide (1) in high-temperature water to obtain a first hydrolysate; and
(ii) a second hydrolysis step of subjecting the first hydrolysate to an enzymatic hydrolysis to obtain a second hydrolysate,
   wherein the first hydrolysate contains a water-soluble polyamide that dissolves in water at 20 °C, and
   the second hydrolysate contains the dicarboxylic acid compound (2) and/or the diamine compound (3), and optionally, the salt compound (4), the polyamide (5), and/or the polyamide (6): 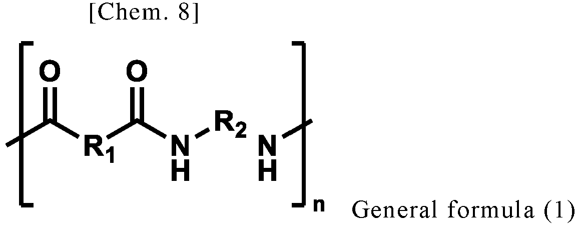 in the formula,
   R₁ is a substituted or unsubstituted aliphatic or aromatic hydrocarbon group having 1 to 10 carbons, and
   R₂ is a substituted or unsubstituted aliphatic hydrocarbon group having 2 to 10 carbons, 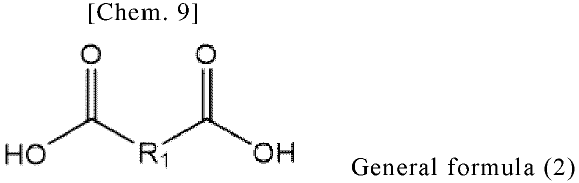 in the formula, R₁ is as defined in the general formula (1). 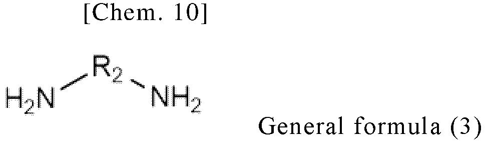 in the formula, R₂ is as defined in the general formula (1), 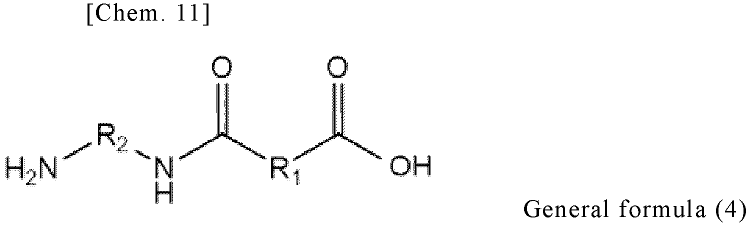 in the formula, R₁ and R₂ are as defined in the general formula (1), 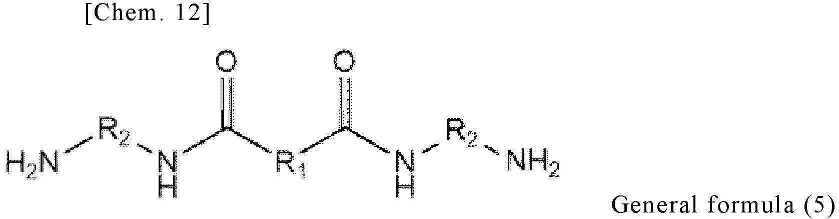 in the formula, R₁ and R₂ are as defined in the general formula (1), 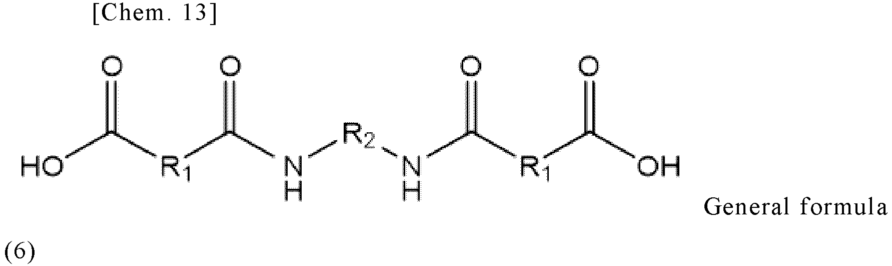 in the formula, R₁ and R₂ are as defined in the general formula (1).

Note that the polyamide (1) represented by the above general formula (1) may be referred to herein simply as "polyamide (1)". The dicarboxylic acid compound (2) represented by the above general formula (2) may be referred to simply as "dicarboxylic acid compound (2)" or "compound (2)". The diamine compound (3) represented by the above general formula (3) may be referred to simply as "diamine compound (3)" or "compound (3)". The salt compound (4) represented by the general formula (4) may be referred to simply as "salt compound (4)" or "compound (4)". The polyamide (5) represented by the above general formula (5) may be referred to simply as "polyamide (5)". The polyamide (6) represented by the above general formula (6) may be referred to simply as "polyamide (6)". The cyclic compound (7) represented by the general formula (7) described below may be referred to simply as "cyclic compound (7)" or "compound (7)".

In one preferred aspect, the dicarboxylic acid compound (2) represented by the general formula (2) and the diamine compound (3) represented by the general formula (3) are produced from the polyamide (1) represented by the general formula (1). The second hydrolysate contains the dicarboxylic acid compound (2) and the diamine compound (3).

In this preferred aspect, in addition to the dicarboxylic acid compound (2) and the diamine compound (3), the salt compound (4) represented by the general formula (4), the polyamide (5) represented by the general formula (5), and/or the polyamide (6) represented by the general formula (6) are also produced. The second hydrolysate may contain the salt compound (4), the polyamide (5), and/or the polyamide (6), in addition to the dicarboxylic acid compound (2) and the diamine compound (3).

### <Polyamide (1)>

The polyamide (1) is n,m-nylon, and examples thereof include, but are not limited to, Nylon 66, Nylon 610, Nylon 6T, Nylon 61, Nylon 9T, and Nylon M5T, for example.

The method for synthesizing the polyamide (1) is not particularly limited. However, it can involve mixing the compound (2), an ester compound of the compound (2) with an alcohol, or an acid halide of the compound (2), or a mixture thereof, with the compound (3). Synthesis is achieved through a condensation reaction involving dehydration, alcohol elimination, and elimination of halide ions, either individually or as a composite reaction.

### <Dicarboxylic acid compound (2) (compound (2))>

The compound (2) has R₁ and two carboxyl groups. In the compound (2), R₁ is as described in R₁ of the polyamide (1).

### <Diamine compound (3) (compound (3))>

The compound (3) has R₂ and two amino groups. In the compound (3), R₂ is as described in R₂ of the polyamide (1).

### <Salt compound (4) (compound (4))>

The compound (4) is a salt (dehydration condensate) of one molecule of the dicarboxylic acid compound (2) and one molecule of the diamine compound (3), having a carboxyl group at one end and an amino group at the other end. In the compound (4), R₁ and R₂ are as described in R₁ and R₂ of the polyamide (1), respectively.

### <Polyamide (5)>

The polyamide (5) is a dehydration condensate of one molecule of the dicarboxylic acid compound (2) and two molecules of the diamine compound (3), with amino groups at both ends. In the polyamide (5), R₁ and R₂ are as described in R₁ and R₂ of the polyamide (1), respectively.

### <Polyamide (6)>

The polyamide (6) is a dehydration condensate of two molecules of the dicarboxylic acid compound (2) and one molecule of the diamine compound (3), with carboxyl groups at both ends. In the polyamide (6), R₁ and R₂ are as described in R₁ and R₂ of the polyamide (1), respectively.

In the general formulas (1) to (6), R₁ is a substituted or unsubstituted aliphatic or aromatic hydrocarbon group having 1 to 10 carbons. Here, aliphatic hydrocarbon groups include chain aliphatic hydrocarbon groups and cyclic aliphatic hydrocarbon groups. Chain aliphatic hydrocarbon groups include linear hydrocarbon groups and branched aliphatic hydrocarbon groups. When R₁ is a substituted aliphatic or aromatic hydrocarbon group, substituents include, for example, methyl, ethyl, n-propyl, and isopropyl groups.

R₁ is preferably an unsubstituted aliphatic hydrocarbon group or aromatic hydrocarbon group having 1 to 10 carbons, more preferably an unsubstituted linear aliphatic or aromatic hydrocarbon group having 4 to 8 carbons, even more preferably an unsubstituted linear aliphatic hydrocarbon group having 4 to 8 carbons, and most preferably an unsubstituted linear aliphatic hydrocarbon group having 4 carbons.

In general formulas (1) to (6), R₂ is a substituted or unsubstituted aliphatic hydrocarbon group having 2 to 10 carbons. Here, aliphatic hydrocarbon groups include chain aliphatic hydrocarbon groups and cyclic aliphatic hydrocarbon groups, and chain aliphatic hydrocarbon groups include linear aliphatic hydrocarbon groups and branched aliphatic hydrocarbon groups, as described above for R₁. When R₂ is a substituted aliphatic hydrocarbon group, substituents include, for example, methyl, ethyl, *n*-propyl, and isopropyl groups.

R₂ is preferably a substituted or unsubstituted aliphatic hydrocarbon group having 5 to 9 carbons, more preferably an unsubstituted aliphatic hydrocarbon group having 5 to 9 carbons, even more preferably an unsubstituted linear aliphatic hydrocarbon group having 6 to 9 carbons, and most preferably an unsubstituted linear aliphatic hydrocarbon group having 6 carbons.

The correspondence among the polyamide (1), the dicarboxylic acid compound (2), and the diamine compound (3), and R₁ and R₂ is exemplified as follows, but are not limited to these.

**[Table 1]**

| R₁ | R₂ | Polyamide (1) | Dicarboxylic acid compound (2) | Diamine compound (3) |
|---|---|---|---|---|
| -(CH₂)₆- | -(CH₂)₆- | Nylon 66 | Adipic acid | Hexamethylenediamine |
| -(CH₂)₁₀- | -(CH₂)₆- | Nylon 610 | Sebacic acid | Hexamethylenediamine |
| -(CH₂)₁₀- | -(CH₂)₆- | Nylon 6T | Terephthalic acid | Hexamethylenediamine |
| -C₆H₆- | -(CH₂)₆- | Nylon 61 | Isophthalic acid | Hexamethylenediamine |
| -C₆H₆- | -(CH₂)₉- | Nylon 9T | Terephthalic acid | Nonanediamine |
| -C₆H₆- | -(CH₂)₃-CH(CH₃)-CH₂- | Nylon M5T | Terephthalic acid | Methylpentanediamine |

### <Step (i): First hydrolysis step>

In this step, the polyamide (1) is hydrolyzed in high-temperature water to obtain the first hydrolysate.

In this step, the polyamide (1) is hydrolyzed to be lower molecular weight components, causing the fixation of oligomers via hydrogen bonds to loosen, resulting in the production of water-soluble polyamide that dissolves in water. Thus, the first hydrolysate contains a water-soluble polyamide. The term "water-soluble polyamide" refers to polyamide that is formed through the reduction of the molecular weight of the polyamide (1) and is soluble in water at 20 °C. The above water-soluble polyamide may contain the salt compound (4), polyamide (5), and/or polyamide (6).

The hydrolysis in the step (i) is also referred to herein as "high-temperature water hydrolysis.

In addition to the above water-soluble polyamide, the first hydrolysate further contains a polyamide that is produced through the reduction of the molecular weight of the polyamide (1) and is not soluble in water at 20 °C. In this specification, a polyamide that does not dissolve in water at 20 °C is also referred to as "water-insoluble polyamide". The number average molecular weight of the water-insoluble polyamide in the first hydrolysate is not particularly limited, but, for example, is 10000 or less, preferably 7000 or less, more preferably 5000 or less, even more preferably 4000 or less, and still even more preferably 3000 or less. If the water-insoluble polyamide has a high molecular weight and has low water solubility, it is less susceptible to the enzymatic hydrolysis reaction. Yet, reducing the average molecular weight improves reactivity with an enzyme in the subsequent second hydrolysis step.

The content of water-insoluble polyamide in the first hydrolysate is not particularly limited, but may be from 1 to 95 weight%, may be from 10 to 90 weight%, or may be from 50 to 85 weight%. In particular, when the content of water-insoluble polyamide is 10 weight% or more, the number average molecular weight of the water-soluble polyamide is reduced relative to the raw material polyamide, and the subsequent second hydrolysis tends to proceed more smoothly.

Although the method is not particularly limited, the molecular weight of water-insoluble polyamide can be measured by GPC, for example. Specifically, the method described in Examples to be described below is exemplified.

In addition to the above water-soluble polyamide and water-insoluble polyamide, the first hydrolysate may further contain the compound (2), the compound (3), a compound (7), which is a decomposed product of the compound (3) as described below, and a compound (8), which is a decomposed product of the compound (2) as described below.

As used herein, "high-temperature water" refers to water with a temperature of 200 °C or higher. High-temperature water includes subcritical and supercritical water. Water during the reaction is liquid or in a supercritical state.

Supercritical water refers to water in a supercritical state. The supercritical state of water is the state in which the temperature is higher than the critical temperature (374 °C) of water and the pressure is higher than the critical pressure (22.1 MPa). In this specification, the critical point is also considered to be included in a supercritical state.

Subcritical water is water in a subcritical state. The subcritical state of water is a state in which the temperature is lower than the critical temperature of the water and the pressure is equal to or higher than the saturated vapor pressure. Thus, the reaction pressure in the step (i) is equal to or higher than the saturated water vapor pressure when the reaction temperature is lower than the critical temperature of the water, and is equal to or higher than the critical pressure when the reaction temperature is higher than the critical temperature of the water.

The reaction temperature in the step (i) is the temperature of high-temperature water, and is, but not particularly limited to, preferably 200 °C or higher and 400 °C or lower, more preferably 230 °C or higher and 350 °C or lower, and even more preferably 230 °C or higher and 300 °C or lower. If the reaction temperature is too low, the hydrolysis reaction of polyamide tends to slow down. If the reaction temperature exceeds 400 °C, the decomposition reaction is more pronouncedly proceed and the yields of the monomers tend to decrease.

In one preferred aspect, the high-temperature water in the step (i) is subcritical water. In other words, in this aspect, the temperature of the high-temperature water in the step (i) is lower than the critical temperature of water. In this case, it is sufficient that the reaction pressure is equal to or higher than the saturated water vapor pressure at the corresponding reaction temperature. Although the upper limit of reaction pressure is not particularly limited, the reaction pressure is preferably equal to or lower than the saturated water vapor pressure + 20 MPa or less, more preferably equal to or lower than the saturated water vapor pressure + 15 MPa, even more preferably equal to or lower than the saturated water vapor pressure + 5 MPa, and most preferably the saturated water vapor pressure. From the viewpoint of selecting an apparatus used for the reaction, the reaction pressure is preferably not too much higher than the saturated water vapor pressure.

The reaction pressure may be set to the saturation vapor pressure by heating in a sealed state, or the vessel may be pressurized by injecting an inert gas into the vessel in a sealed state. Pressurization with the inert gas may be done before or after heating.

When the reaction temperature in the step (i) is equal to or higher than the critical temperature of water, it is sufficient that the reaction pressure is equal to or higher than the critical pressure. Although the upper limit of the reaction pressure is not particularly limited, the reaction pressure is preferably equal to or lower than the critical pressure + 15 MPa, more preferably equal to or lower than the critical pressure + 5 MPa, and particularly preferably the critical pressure. From the viewpoint of selecting an apparatus used for the reaction, the reaction pressure preferably is not too much higher than the critical pressure.

In the step (i), if the temperature of the high-temperature water is equal to or higher than the critical temperature, corrosiveness significantly improves, raising the possibility of metal contamination from the apparatus and degradation of the apparatus due to corrosion.

Stirring during the reaction is not necessarily required, but stirring is preferred. By stirring, the surface renewal of the polyamide solid is achieved, leading to uniform progress of the reaction and an improvement in the reaction rate.

The shape of the reactor is not particularly limited, and reactors in any shape, such as a tank or circulation type, can be used.

The reaction time is not particularly limited, but is preferably from 5 to 300 minutes, more preferably from 5 to 100 minutes, and even more preferably from 5 to 30 minutes. The reaction time shall be measured from the time the desired reaction conditions are reached until the heating is stopped. If the reaction time is short, high molecular weight components that cannot react with an enzyme may remain in a higher content, whereas if the reaction time is too long, decomposition reactions proceed, leading to a significant decrease in yields.

The concentration of polyamide in water (high-temperature water) is not particularly limited, but is preferably from 1 to 50 weight%, more preferably from 5 to 40 weight%, and most preferably from 7 to 30 weight%. If the concentration is too low, recovery becomes extremely difficult, whereas if the concentration is too high, the fluidity of the reaction solution decreases, making it difficult to achieve a uniform reaction.

To improve the reaction efficiency of the first hydrolysis step in high-temperature water, the polyamide (1) may also be pretreated.

For example, it can be microparticulated to ensure uniformity of the reaction in the first hydrolysis step. The method for microparticulation is not particularly limited, but is exemplified by the method in which the polyamide (1) is dissolved in a solvent and then recrystallized, re-precipitated, or spraydried, for example, is used, as well as the microparticulation method by milling, such as freezing and pulverizing. The method by dissolving the polyamide (1) and then microparticulating the resultant is preferred because it enables uniform microparticulation regardless of the shape of the polyamide (1) used as the raw material.

Alternatively, to improve reactivity after the reaction has started, sufficient water may be permeated into the polyamide (1) in advance. The permeation method is not particularly limited and is exemplified by, for example, the method in which the polyamide (1) is immersed in water followed by heating, the method in which the polyamide (1) is exposed to a humidified atmosphere, and the method in which the polyamide (1) is precipitated after it is dissolved in a solvent and water is added.

A catalyst may also be pre-mixed so that the reaction starts uniformly at the beginning of the reaction. The catalyst to be pre-mixed is not particularly limited, but examples thereof include acidic compounds, basic compounds, and inorganic salts, for example. Inorganic salts are preferred as the catalyst to be pre-mixed in view of their separability from the obtained compound (2) and compound (3), etc., and their use as raw materials for polymerization.

The method for mixing the catalyst is also not particularly limited, and is exemplified by the method in which the polyamide (1) is immersed in a solvent in which the catalyst to be mixed has been dissolved, the method in which the polyamide (1) and the catalyst to be mixed is of melt kneaded, and the method in which the polyamide (1) and the catalyst to be mixed are dissolved in a solvent and mixed and then the polyamide (1) is precipitated, for example.

In one aspect, the first hydrolysate further includes the monomers and decomposed products thereof. Specifically, the first hydrolysate further contains the compound (3) and a cyclic compound (7) (cyclization product of diamine) represented by the following general formula (7). The compound (7) is a compound formed through the decomposition of the compound (3). Here, the decomposition is specifically due to the cyclization reaction of the compound (3): 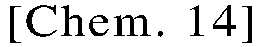 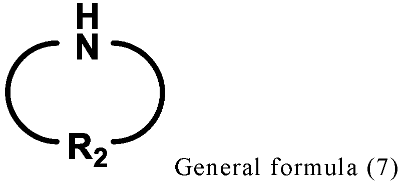 in the formula, R₂ is as defined in the general formula (1).

Examples of the compound (7) include, for example, hexamethyleneimine, nonamethyleneimine, and 4-methylazepane. When the compound (3) is hexamethylenediamine, the compound (7) is hexamethyleneimine.

There is no particular limitation on the amount of monomer and decomposed products thereof after the first hydrolysis step. However, in view of improving the reaction yields of the target products, *i.e*., the compound (2) and compound (3), after the enzymatic hydrolysis step, in other words, the reaction yields of the target products by the subsequent second hydrolysis step, it is preferable that the sum of the yields of the compound (3) and compound (7) in the first hydrolysate is preferably 20% or less. The sum of the yields is more preferably from 0.1 to 20%, even more preferably from 0.5 to 15%, and still even more preferably from 0.8 to 12%. The compound (7) is produced through the decomposition of compound (3). Thus, if the sum of the yields of compound (3) and compound (7) is too high, it indicates that not only the hydrolysis reaction but also the decomposition reaction is proceeding. This may lead to a lower yield after the enzymatic hydrolysis step. Otherwise, if the sum of the yields is too low, it suggests that the molecular weight of the polymer has not reduced sufficiently, making it difficult for the enzymatic hydrolysis reaction to proceed.

The first hydrolysate may further contain a compound (8) (cyclization product of the dicarboxylic acid), which is a decomposed product of the compound (2). Specifically, the compound (8), which is a decomposed product of the compound (2), is formed by the cyclization reaction of the compound (2). The compound (8), which is a decomposed product of the compound (2), is, for example, cyclopentanone when the compound (2) is adipic acid.

There is no particular limitation on the amount of monomers and decomposed products thereof after the first hydrolysis step. However, in view of improving the reaction yields of the target products, *i.e*., the compound (2) and compound (3), after the enzymatic hydrolysis step, in other words, the reaction yields of the target products by the subsequent second hydrolysis step, the sum of the yields of the compound (2) and the compound (8) in the first hydrolysate may be from 0.1% to 40%, may be from 0.5% to 20%, or may be from 0.8% to 10%. The compound (8) is produced through the decomposition of the compound (2). Thus, if the sum of the yields of compound (2) and compound (8) is too high, it indicates that not only the hydrolysis reaction but also the decomposition reaction is proceeding. This may lead to a lower yield after the enzymatic hydrolysis step. Otherwise, if the sum of the yields is too low, it suggests that the molecular weight of the polymer has not reduced sufficiently, making it difficult for the enzymatic hydrolysis reaction to proceed.

There is no particular limitation on the amount of monomers and decomposed products thereof after the first hydrolysis step. However, in view of improving the reaction yields of the target products, *i.e*., the compound (2) and compound (3), after the enzymatic hydrolysis step, in other words, the reaction yields of the target products by the subsequent second hydrolysis step, the sum of the yields of the compound (7) and compound (8) in the first hydrolysate may be from 0% to 80%, may be from 0.1% to 60%, or may be from 0.5% to 20%. The compound (7) and compound (8) are produced through the decomposition of the compound (3) and compound (2), respectively. Thus, if the sum of the yields of compound (7) and compound (8) is too high, it indicates that not only the hydrolysis reaction but also the decomposition reaction is proceeding. This may lead to a lower yield after the enzymatic hydrolysis step. Otherwise, if the sum of the yields is too low, it suggests that the molecular weight of the polymer has not reduced sufficiently, making it difficult for the enzymatic hydrolysis reaction to proceed.

When the compound (7), which is a decomposed product of the compound (3), and the compound (8), which is a decomposed product of the compound (2), are formed by the first hydrolysis step, the pH of the hydrolysis solution changes. Therefore, the progress of the reaction can be checked by measuring the pH of the hydrolysis reaction solution. There is no particular limitation on the pH of the hydrolysis reaction solution at the end of the first hydrolysis step. However, if the reaction is started when the concentration of the polyamide (1) (initial concentration of the polyamide (1)) is 10 weight% relative to 100 weight% of water (high-temperature water), it is preferable to stop the reaction at a pH of 7.0 to 10.0, it is more preferable to stop the reaction at a pH of 8.0 to 10.0, and it is even more preferable to stop the reaction at a pH of 9.0 to 9.5. Since a pH of 10.0 or lower can prevent the deactivation of the enzyme, the subsequent secondary hydrolysis tends to proceed favorably.

Furthermore, if the concentration of the charged polyamide (1) is other than 10 weight%, for example, a sample for pH measurement sampled from the reaction solution is concentrated or diluted with water at the concentration ratio or dilution ratio so that the concentration of the charged polyamide (1) becomes 10 weight%. Thereafter, the pH can be measured to determine and adjust the progress of the reaction.

Methods for concentration and measurement of the pH are not particularly limited and generally-used methods can be used. Specifically, the methods described in Examples to be described below are exemplified.

The solution after the end of the reaction may be post-treated as needed, and the method used in the post-treatment is not particularly limited.

The pH after the first hydrolysis reaction may be adjusted by adding an acidic or basic compound to a range suitable for the subsequent second hydrolysis step by the enzyme, and/or a buffer solution may be used to reduce fluctuations of the pH.

The concentrations of the polyamide (1), compound (2), compound (3), and water-soluble polyamide (which may include the salt compound (4) and polyamides (5) and/or (6)) may be adjusted by concentration or dilution with water as needed to a level suitable for carrying out the subsequent second hydrolysis step.

Solid-liquid separation may be performed on the solid component (water-insoluble polyamide) precipitated by the first hydrolysis step, or the solid component may be fed directly to the second hydrolysis step. When solid-liquid separation is performed, only the soluble components are hydrolyzed by the enzyme, and thus the reaction is completed rapidly. On the other hand, when no separation is performed, enzymatic hydrolysis of the solid polymer with a reduced molecular weight also proceeds, resulting in higher yields of the compound (2) and compound (3).

### <Step (ii): Second hydrolysis step>

In this step, the first hydrolysate is subjected to enzymatic hydrolysis to obtain the second hydrolysate. Specifically, the polyamide (1) of which molecular weight has been reduced in the step (i) is subjected to an enzymatic hydrolysis reaction to obtain the dicarboxylic acid compound (2) and/or the diamine compound (3) and, optionally, the salt compound (4), the polyamide (5), and/or the polyamide (6). Thus, the second hydrolysate contains the dicarboxylic acid compound (2) and/or the diamine compound (3) and, optionally, the salt compound (4), the polyamide (5), and/or the polyamide (6).

More specifically, the second hydrolysate includes water-soluble polyamides (such as the salt compound (4), polyamides (5) and/or (6)), the water-insoluble polyamide, the dicarboxylic acid compound (2), the diamine compound (3), the compound (7), which are decomposed products of the diamine compound (3), and/or the compound (8), which is a decomposed product of the compound (2).

The enzymes that can be used for the hydrolysis of polyamide in this step are not particularly limited as long as it has hydrolytic activity, but examples thereof include NylA (EC 3.5.2.12), which is a degrading enzyme for the cyclic dimer of 6-aminocaproic acid; NylB (EC 3.5.1.46), which is an exo-type oligomerase; and NylC (EC 3.5.1.117), which is an endo-type oligomerase. Specific examples of typical enzymes include, but are not limited to, NylA, NylB, and NylC derived from *Arthrobacter sp.* KI72; NylA and NylB derived from *Pseudomonas sp.* NK8; NylB and NylC derived from *Agromyces sp.* KY5R; and NylA, NylB, and NylC derived from *Kocuria sp.* KY2.

Furthermore, one enzyme may be used or a combination of several enzymes may be used in this step.

The amino acid sequences of the enzymes mentioned above which can be used in the present disclosure may have any mutations that can occur naturally and/or any mutations and modifications that can be artificially introduced, as long as they exhibit enzyme activity. For example, mutations such as deletions, substitutions, insertions, and additions may be included. For example, the amino acid sequences of the above enzymes may contain one or more, preferably from 1 to 20, more preferably from 1 to 10, even more preferably from 1 to 7, particularly preferably from 1 to 5, further particularly preferably from 1 to 3 deletions, substitutions, insertions, and/or additions.

The production method of the enzyme is exemplified by, for example, the method of producing the enzymes using an organism that originally produces the enzyme and the method of producing the enzymes in a host cell by genetic recombination, but is not limited to these methods as long as the enzyme with activity can be produced. Examples of the host organism for heterologous expression include, but are not limited to, bacteria such as *Escherichia coli, Corynebacterium, Bacillus subtilis, Brevibacillus brevis,* and *Rhodococcus,* fungi such as budding yeast, fission yeast, Pichia yeast, and *Aspergillus,* as well as eukaryotic cells such as insect cells, animal cells, and plant cells.

The form of enzyme used in the reaction is not particularly limited. The enzyme produced by biological methods may be used in as it is, including the enzyme contained within cells or extracellular sources (such as culture media). Additionally, the enzyme may be used after being treated, such as separated, purified, or concentrated from cells or extracellular sources (such as culture media). Known methods can be used for treatment, such as the addition of lysozyme and surfactants, ultrasonication, cell disruption methods like French press, washing, centrifugation, various chromatography techniques, various membrane separations, salting out, solvent precipitation, dialysis, and electrophoresis. These methods can be selected or combined as appropriate. Alternatively, the enzyme may be immobilized by inclusion, cross-linking, or carrier-binding methods from the viewpoint of reuse of the enzyme. Examples of immobilization carriers for immobilization include, but are not limited to, glass beads, silica gel, polyurethane, polyacrylamide, polyvinyl alcohol, carrageenan, and alginic acid.

The temperature of the enzymatic reaction in the second hydrolysis step is not particularly limited, but is, for example, from 10 to 90 °C, preferably from 20 to 80 °C. If the reaction temperature is too low, the reaction rate is low, whereas if the reaction temperature is too high, the enzyme is more likely to be inactivated. The reaction pH may be adjusted by adding an acidic or basic compound, and/or a buffer solution may be used to reduce fluctuations of the pH. The PH is preferably from 2.0 to 10.0, and more preferably from 3.0 to 8.5. The reaction time depends on the amount of enzyme used, but is preferably from 20 minutes to 200 hours, more preferably from 6 to 80 hours in view of industrial application. However, the present disclosure is not particularly limited to the reaction conditions and reaction mode described above, and the reaction conditions and reaction mode may be selected as appropriate.

The method for recovery of the target products, namely, the compound (2) and/or compound (3), and the optional target products, namely, the salt compound (4), polyamide (5) and/or polyamide (6), is not particularly limited, but all of these may be recovered together or each of these may be recovered individually. For example, the compound (2) and compound (3) may be recovered as their salts (in the form of salt compounds (4)).

Methods for individual recovery are exemplified by the crystallization method by utilizing the difference in solubility or boiling point of each compound, and the separation and recovery method by distillation. For example, the compound (2) and polyamide (6) can be caused to be precipitated by lowering the pH by adding an acidic compound to the reaction solution, and the precipitated compound (2) and polyamide (6) can be separated and recovered by crystallization. A basic compound can be added to the remaining solution from which the compound (2) and polyamide (6) have been removed, to raise the pH. Subsequently, the compound (3) and polyamide (5) can be separated and recovered by distillation.

The method for recovering the salt compound (4) and the method for recovering the compound (2) and compound (3) by converting them into respective salt compounds (4) is exemplified by the method in which the reaction solution is first subjected to concentration to dryness, reprecipitation or recrystallization in the aforementioned separation and recovery method. Recrystallization is preferred from the viewpoint of removing impurities.

The recrystallization method is exemplified by, for example, the method by concentrating the reaction solution, the method by cooling the reaction solution, and the method by adding a poor solvent of the salt to the reaction solution. The recrystallization method is preferred from the viewpoint of improving yield and reducing the amount of impurities remaining.

Other separation and recovery methods include the method by ultrafiltration or nanofiltration, which uses sieving based on molecular weight.

The target products, *i.e.,* the compound (2) and/or compound (3), obtained by the production method of the present disclosure, and the optional target products, *i.e*., the salt compound (4), polyamide (5) and/or polyamide (6), may be reused as raw materials for the polymerization of a polyamide in the state of mixture. Alternatively, only the mixture of the compound (2) and/or the compound (3) and salts thereof, *i.e*., the salt compounds (4), may be reused as raw materials for the polymerization of a polyamide. Alternatively, only the compound (2) and/or the compound (3) may be reused as raw materials for the polymerization of a polyamide.

As described above, in accordance with the present disclosure, a novel method of producing a dicarboxylic acid compound and/or a diamine compound, which are raw materials for the polymerization of a polyamide useful as engineering plastics, from a polyamide, may be provided. In the production method in accordance with the present disclosure, a dicarboxylic acid compound and/or a diamine compound can be produced from a polyamide, particularly m,n-nylon, in high yields by combining hydrolysis in high-temperature water and enzymatic hydrolysis. Here, the combination with enzymatic reaction can also suppress the decomposition reaction by cyclization, which further improves the yields. According to the production method in accordance with the present disclosure, the addition of acidic compounds (such as formic acid) for the purpose of reducing molecular weight is not necessary. Therefore, acidic compounds or their derivatives are mixed into the reaction system or the target products during production. This allows for obtaining the target products with high purity. Additionally, there is no need for a separation step to remove the acidic compounds.

### EXAMPLES

While the present disclosure will now be described in more detail with reference to examples and comparative examples, it is understood that the present disclosure is not particularly limited to these examples.

The measurement and analytical methods used in examples and comparative examples are as follows:
(1) Liquid chromatograph (LC)
   Purpose of analysis: Quantification of dicarboxylic acid compound (2)
   Measurement apparatus: Prominence (manufactured by Shimadzu Corporation)
   Mobile phase: 8 mM methanesulfonic acid
   Column: Shim-pack Fast-OA (G) column + 2 Shim-pack Fast-OA columns (7.8 × 100 mm)
   Column temperature: 35 °C
   Flow rate: 0.6 mL/min
   Injection amount: 10 µL
   Detector: Differential refractive index detector, UV detector at 210 nm
(2) Gas chromatograph (GC)
   Purpose of analysis: Quantification of cyclic compound (8) of dicarboxylic acid
   Measurement apparatus: GC-2030
   Carrier gas: He
   Column: DB-1 30 m × 0.25 mm × 1.0 um (manufactured by Agilent Technologies)
   Detector: FID
   Injection amount: 1.0 µL
   Split ratio: 1:40
   Column temperature: The temperature was held at 45 °C for 5 min, then increased at 20 °C/min, and held at 300 °C for 20 min
   Injection temperature: 250 °C
   Detector temperature: 300 °C
(3) Ion chromatograph (IC)
   Purpose of analysis: Quantification of diamine (3) and cyclization product of diamine (7)
   Measurement apparatus: Integrion RFIC (manufactured by Thermo Fisher Scientific)
   Mobile phase: 0 to 0.1 min - methanesulfonic acid solution at 9 mM
   0.1 to 10.7 min - methanesulfonic acid solution from 9 mM to 65 mM
   10.7 to 12.8 min - methanesulfonic acid solution at 65 mM
   Column: IonPac CG19 (2 × 50 mm) / CS19 (2 × 250 mm) (manufactured by Thermo Fisher Scientific)
   Column temperature: 30 °C
   Flow rate: 0.325 mL/min
   Injection amount: 25 µL
   Detector: Conductivity detector
(4) Size-exclusion chromatography (SEC)
   Purpose of analysis: Determination of molecular weights
   Measurement apparatus: TOSOH HLC-8320 GPC
   Mobile phase: HFIP (Sodium trifluoroacetic acid, 5 mmol/L)
   Column : 3 TSKgel GMHHR-H (S) columns (4.6 mm I.D. × 15 cm)
   Column temperature: 40 °C
   Flow rate: 0.175 ml/min
   Injection amount: 10 µL
   Standard sample: PMMA
   Detector: Differential refractive index analysis
(5) pH
   The pH of each solution obtained in Examples 1 to 5 and Comparative Examples 1 to 3 is measured with a portable pH meter D-51S manufactured by Horiba, Ltd.

### <Construction of plasmid for enzyme expression>

KOD Plus Neo (product name, manufactured by Toyobo Co., Ltd.) was used for the amplification of PCR fragments, and *E. coli* JM109 strain (manufactured by NIPPON GENE CO., LTD.) was used for the preparation of plasmids. The artificial gene synthesis service available from Eurofins Genomics was used to optimize the base sequences.

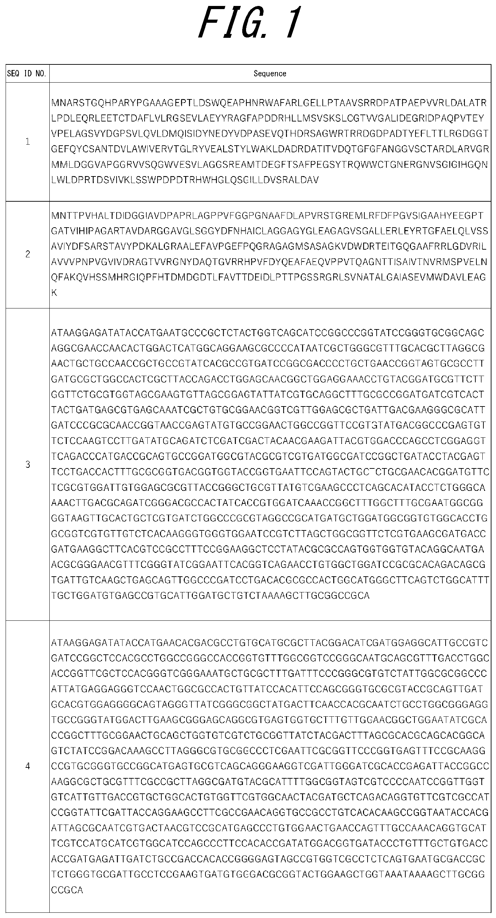

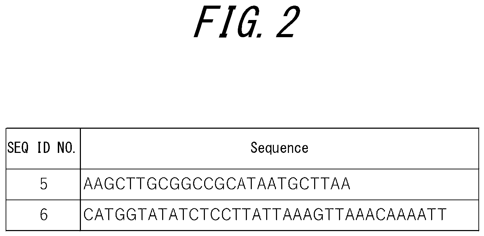

The DNA fragment containing the coding region of NylB protein (SEQ ID NO. 1) derived from *Arthrobacter sp.* KI72 and the DNA fragment containing the coding region of NylC (m) protein (SEQ ID NO. 2) which is a variant of NylC protein derived from *Arthrobacter sp.* KI72 were each codon-optimized for expression in *E. coli,* and synthesized using the artificial gene synthesis service available from Eurofins Genomics (SEQ ID NOS. 3 and 4) (FIG. 1). PCR was performed using pRSFDuet-1 (product name, manufactured by Merck) as a template and oligonucleotides of SEQ ID NOS. 5 and 6 (FIG. 2) as primers to obtain the pRSFDuet-1 fragment. The DNA fragment containing the gene coding region was connected to the pRSFDuet-1 fragment using an In-Fusion HD cloning kit (product name, manufactured by Clontech). *E. coli* JM109 strain was transformed by the plasmid and the plasmid was extracted from the resulting transformant. The plasmid for NylB expression "nylB-pRSFDuet" and the plasmid for NylC(m) expression "nylC(m)-pRSFDuet" were obtained.

### <Preparation of a crude enzyme solution>

Competent cells of *E. coli* BL21 (DE3) strain (manufactured by Nippon Gene Co., Ltd.) were transformed by the constructed plasmids. The transformed cells were then spread onto LB agar media (10 g/L of tryptone, 5 g/L of yeast extract, 5 g/L of sodium chloride, and 15 g/L of agar) containing 100 mg/L of kanamycin sulfate. The media were incubated at 37 °C for 24 hours to obtain single colonies of transformants. One platinum loop of the transformants was each inoculated into 2 mL of LB liquid medium (10 g/L of tryptone, 5 g/L of yeast extract, and 5 g/L of sodium chloride) containing 50 mg/L of kanamycin sulfate (in a 14 mL round-bottom tube). The cultures were shaken at 37 °C for 24 hours to obtain pre-cultures. The obtained pre-cultures (500 µL) were each inoculated into 50 mL of LB liquid medium containing 50 mg/L of kanamycin sulfate (in a 100 mL flask). The cultures were shaken at 37 °C. After 3 hours of cultivation, isopropyl-β-thiogalactopyranoside (IPTG) was added to a final concentration of 0.1 mM. The cultures were then shaken at 30 °C for 24 hours. After centrifuging 45 mL of the resulting culture medium at 4000 × g for 5 minutes, the supernatant was removed to obtain pellets. After washing the pellets with 10 mL of a 100-mM Tris hydrochloric acid buffer (pH: 7.5), the pellets were centrifuged at 4000 × g for 5 minutes and the supernatant was removed. The pellets were suspended in 2 mL of a 100-mM Tris hydrochloric acid buffer (pH: 7.5) and the cells were disrupted in an ultrasonic disruption machine (Digital Sonifier 450, product name, manufactured by Branson). The disrupted solution was centrifuged at 4000 × g for 5 minutes, to obtain a crude enzyme solution containing NylB or a crude enzyme solution containing NylC (m).

### [Example 1]

A stationary pressure vessel made of Inconel with an inner diameter of 30 mm and a depth of 78 m was charged with 3 g of Nylon 6,6 (polycondensate of adipic acid and hexamethylenediamine, number average molecular weight: 23000) and 30 g of water and sealed. Nitrogen was circulated through the vessel to replace the gas phase components with nitrogen. The vessel was mounted in an electric furnace and heating was started. The internal temperature was raised to 280 °C and the pressurized with nitrogen was performed. The final temperature and pressure were 280 °C and 22 MPa, respectively. The reaction was caused to take place for 30 minutes in this state. Thereafter, the electric furnace was turned off, and the vessel was removed from the electric furnace and air cooled. After cooling, the treated solution was taken out into a glass bottle and weighed. The solution taken out weighed 30.39 g.

The results of the analyses of the taken-out solution are summarized in Table 2.

### [Examples 2 to 8]

Hydrolysis of Nylon 6,6 was performed in the same manner as in Example 1, except that the internal temperature and pressure of the vessel were changed as summarized in Table 2.

The results of the analyses of the taken-out solutions are summarized in Table 2.

Note that Examples 1 to 5 and 8 were performed in subcritical states (in which the temperature was lower than the critical temperature and the pressure was equal to or higher than the saturated vapor pressure of water), and Examples 6 and 7 were performed in supercritical states (in which the temperature was equal to or higher than the critical temperature and the pressure was equal to or higher the critical pressure of water).

**[Table 2]**

| | | Example 1 | Example 2 | Example 3 | Example 4 | Example 5 | Example 6 | Example 7 | Example 8 |
|---|---|---|---|---|---|---|---|---|---|
| Internal temperature of the vessel (processing temperature) | | 280°C | 250°C | 280°C | 310°C | 340°C | 380°C | 380°C | 220°C |
| Pressure in the vessel (processing pressure) | | 22 MPa | 4.0 MPa | 6.4 MPa | 9.9 MPa | 14.6 MPa | 22 MPa | 28 MPa | 2.3 MPa |
| Processing time | | 30 min. | 30 min. | 30 min. | 30 min. | 30 min. | 30 min. | 30 min. | 30 min. |
| pH after proces sing | | 8.5 | 7.6 | 8.4 | 9.1 | 9.2 | 12.4 | 12.1 | 7.7 |
| Insoluble componen t | Ratio *¹ of water-insoluble polyamide | 48 wt % | 85 wt % | 51 wt % | 25 wt % | 1 wt % | 0 wt % | 0 wt % | 97 wt % |
| | Number average molecular weight of water-insoluble polyamide | 2,800 | 4,410 | 2,780 | 2,380 | 1,180 | - | - | 10,720 |
| Soluble componen t | (2) Yield of adipic acid (ADA) | 6.9% | 1.2% | 5.6% | 7.8% | 10.1% | 7.0% | 8.5% | 0.1% |
| | (3) Yield of hexamethylenediamine (HMD) | 3.7% | 1.0% | 4.1% | 5.2% | 1.9% | 0.0% | 0.0% | 0.0% |
| | (8) Yield of cyclopentanone (CP) | 0.3% | 0.0% | 0.2% | 2.4% | 9.6% | 33.0% | 6.0% | 0.1% |
| | (7) Yield of hexamethyleneimine (HMI) | 0.5% | ND | 0.3% | 2.4% | 9.3% | 40.0% | 52.0% | ND |
| | Ratio *² of water-soluble polyamides | 43 wt % | 13 wt % | 33 wt % | 55 wt % | 73 wt % | 30 wt % | 0 wt % | 4 wt % |
| | Sum of yields of (3) HMD and (7) HMI | 4.2% | 1.0% | 4.4% | 7.6% | 11.2% | 40.0% | 52.0% | 0.0% |
| | Sum of yields of (2) ADA and (8) CP | 7.2% | 1.2% | 5.8% | 10.2% | 19.7% | 40.0% | 14.5% | 0.2% |
| | Sumof yields of (7) HMI and (8) CP | 0.8% | 0.0% | 0.5% | 4.8% | 18.9% | 73.0% | 58.0% | 0.1% |

| | | | | | | | | | |
|---|---|---|---|---|---|---|---|---|---|
| *1 Indicates the ratio of water-insoluble polyamide when nylon 6,6 is regarded as 100 wt %. *2 Indicates the ratio of water-soluble polyamide when nylon 6,6 is regarded as 100 wt %. | | | | | | | | | |

### [Example 9]

To the high-temperature water-treated solution obtaind in Example 1, 34.1 mL of the crude enzyme solution containing Ny1B, 34.1 mL of the crude enzyme solution containing Ny1C(m), 15.7 mL 1-M Tris hydrochloric acid buffer (pH: 7.5), and 42.8 mL of water were added, and the enzymatic reaction was caused to take place at 40 °C for 48 hours. Analysis of the enzymatic reaction mixture revealed the presence of 1.26 g of adipic acid (8.6 mmol, yield: 65%) and 0.92 g of hexamethylenediamine (7.9 mmol, yield: 60%). In addition, H₂N-(CH₂)₆-NH-CO-(CH₂)₄-COOH (hexamethylenediamine adipate), H₂N-(CH₂)₆-NH-CO-(CH₂)₄-CO-NH-(CH₂)₆-NH₂, and COOH-(CH₂)₄-CO-NH-(CH₂)₆-NH-CO-(CH₂)₄-COOH were also present.

The results of the analysis of the enzymatic reaction solution are summarized in Table 3.

### [Examples 10 to 14]

Examples 10 to 14 were performed in the same manner as Example 9, except that the solutions treated with high-temperature water obtained in Examples 2 to 6 were used, respectively, and the enzymatic reaction was caused to take place.

The results of the analysis of the enzymatic reaction solution are summarized in Table 3.

**[Table 3]**

| | Example 9 | Example 10 | Example 11 | Example 12 | Example 13 | Example 14 |
|---|---|---|---|---|---|---|
| (2) Adipic acid | 1.26 g | 0.25 g | 1.11 g | 1.45 g | 1.72 g | 0.42 g |
| | Yield: 65% | Yield: 12% | Yield: 53% | Yield: 73% | Yield: 85% | Yield: 20% |
| (3) Hexamethylenediamine | 0.92 g | 0.25 g | 1.05 g | 1.31 g | 0.45 g | 0.25 g |
| | Yield: 60% | Yield: 16% | Yield: 68% | Yield: 82% | Yield: 30% | Yield: 16% |
| (4) Presence or absence of H₂N-(CH₂)₆-NH-CO-(CH₂)₄-COOH | Present | Present | Present | Present | Present | Present |
| (5) Presence or absence of H₂N-(CH₂)₆-NH-CO-(CH₂)₄-CO-NH-(CH₂)₆-NH₂ | Present | Present | Present | Present | Present | Present |
| (6) Presence or absence of COOH-(CH₂)₄-CO-NH-(CH₂)₆-NH-CO-(CH2)₄-COOH | Present | Present | Present | Present | Present | Present |

### [Comparative Example 1]

To 3 g of Nylon 6,6 that was also used in Example 1, 20 mL of a 1-M Tris hydrochloric acid buffer (pH: 7.5), 20 mL of the crude enzyme solution containing Ny1B, 20 mL of the crude enzyme solution containing Ny1C (m), and 140 mL of water were mixed. The mixture was then subjected to enzymatic reaction at 40 °C for 48 hours. Analysis of the enzymatic reaction mixture revealed the absence of adipic acid hexamethylenediamine.

### INDUSTRIAL APPLICABILITY

In accordance with the present disclosure, a diamine compound and a dicarboxylic acid compound that can be used as raw materials for polyamides and other materials can be produced with a favorable yield.

## Claims

1. A production method of a dicarboxylic acid compound (2) represented by the following general formula (2) and/or a diamine compound (3) represented by the following general formula (3), and optionally, a salt compound (4) represented by the following general formula (4), a polyamide (5) represented by the following general formula (5), and/or a polyamide (6) represented by the following general formula (6), from a polyamide (1) represented by the following general formula (1), the method comprising:
(i) a first hydrolysis step of hydrolyzing the polyamide (1) in high-temperature water to obtain a first hydrolysate; and
(ii) a second hydrolysis step of subjecting the first hydrolysate to an enzymatic hydrolysis to obtain a second hydrolysate,
wherein the first hydrolysate contains a water-soluble polyamide that dissolves in water at 20 °C, and
the second hydrolysate contains the dicarboxylic acid compound (2) and/or the diamine compound (3) and, optionally, the salt compound (4), the polyamide (5), and/or the polyamide (6): 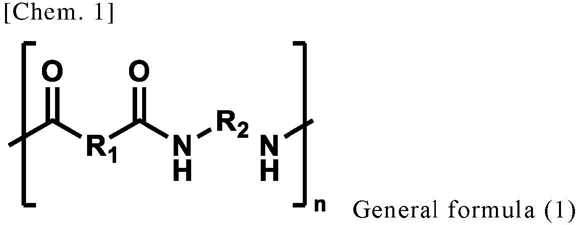 in the formula,
R₁ is a substituted or unsubstituted aliphatic or aromatic hydrocarbon group having 1 to 10 carbons, and
R₂ is a substituted or unsubstituted aliphatic hydrocarbon group having 2 to 10 carbons, 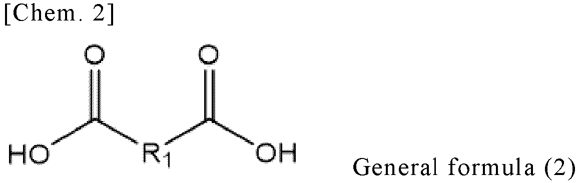 in the formula, R₁ is as defined in the general formula (1), 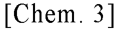 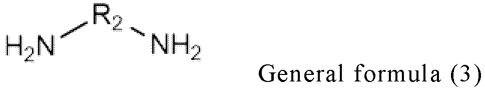 in the formula, R₂ is as defined in the general formula (1), 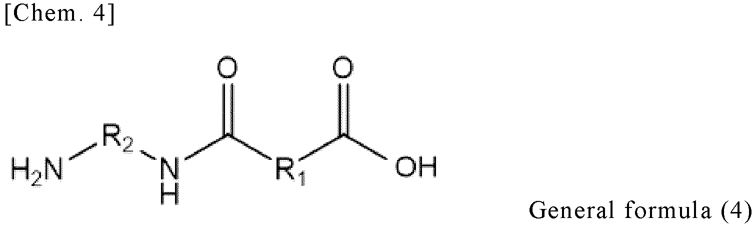 in the formula, R₁ and R₂ are as defined in the general formula (1), 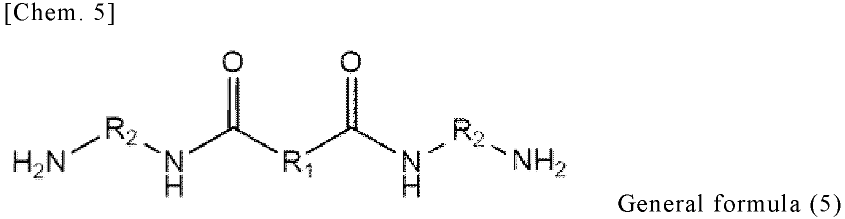 in the formula, R₁ and R₂ are as defined in the general formula (1), 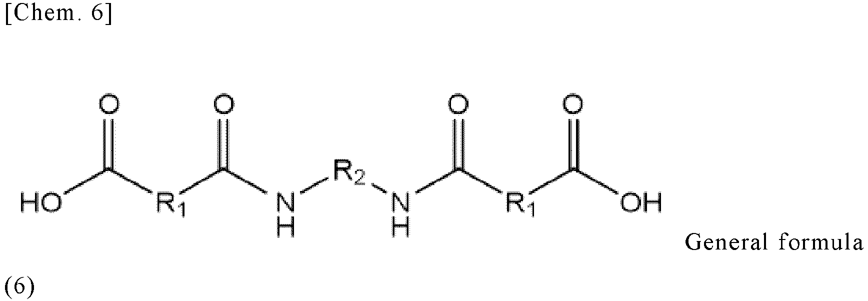 in the formula, R₁ and R₂ are as defined in the general formula (1).

2. The production method according to claim 1, wherein
the dicarboxylic acid compound (2) represented by the general formula (2) and the diamine compound (3) represented by the general formula (3) are produced from the polyamide (1) represented by the general formula (1), and
the second hydrolysate contains the dicarboxylic acid compound (2) and the diamine compound (3).

3. The production method according to claim 1 or 2, wherein a temperature of the high-temperature water is lower than a critical temperature of the water.

4. The production method according to claim 1 or 2, wherein in the first hydrolysis step, a pH of a hydrolysis reaction solution at the end of hydrolysis is from 7.0 to 10.0 when a concentration of the polyamide (1) represented by the general formula (1) is 10 weight% relative to 100 weight% of the high-temperature water.

5. The production method according to claim 1 or 2, wherein
the first hydrolysate further contains a polyamide that is insoluble in water at 20 °C, and
a number average molecular weight of the insoluble polyamide is 5000 or less.

6. The production method according to claim 1 or 2, wherein
the first hydrolysate further contains the diamine compound (3) represented by the general formula (3) and a cyclic compound (7) represented by the following general formula (7), and
a sum of yields of the diamine compound (3) represented by the general formula (3) and the cyclic compound (7) represented by the following general formula (7) is 20% or less: 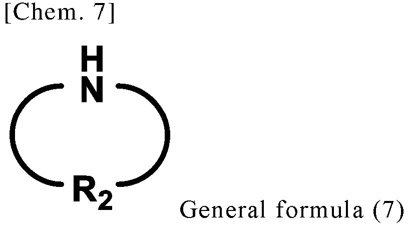 in the formula, R₂ is as defined in the general formula (1).

7. The production method according to claim 1 or 2, wherein R₂ is a substituted or unsubstituted aliphatic hydrocarbon group having 5 to 9 carbons.

8. The production method according to claim 1 or 2, wherein R₁ is an unsubstituted linear aliphatic or aromatic hydrocarbon group having 4 to 8 carbons.

9. The production method according to claim 1 or 2, wherein R₂ is an unsubstituted linear aliphatic hydrocarbon group having 6 carbons.

10. The production method according to claim 1 or 2, wherein R₁ is an unsubstituted linear aliphatic hydrocarbon group having 4 carbons.
